(19) **Europäisches Patentamt European Patent Office Office européen des brevets**

(11) **EP 2 234 673 B1**

(12) **EUROPEAN PATENT SPECIFICATION**

(45) Date of publication and mention
of the grant of the patent:
**27.09.2017 Bulletin 2017/39**

(51) Int Cl.:
*A61Q 5/10* (2006.01)          *A61K 8/73* (2006.01)
*A61K 8/81* (2006.01)          *A61K 8/86* (2006.01)
*A61K 8/89* (2006.01)          *A61Q 5/00* (2006.01)
*A61K 8/898* (2006.01)

(21) Application number: **08866160.8**

(22) Date of filing: **17.12.2008**

(86) International application number:
**PCT/US2008/087188**

(87) International publication number:
**WO 2009/085838 (09.07.2009 Gazette 2009/28)**

(54) **METHOD OF PROTECTING DYED HAIR COLOR FROM FADING OR WASH-OUT**

VERFAHREN ZUM SCHUTZ VON COLORIERTEM HAAR VOR AUSBLEICHUNG ODER
AUSWASCHUNG

PROCÉDÉ DE PROTECTION D'UNE TEINTURE POUR CHEVEUX CONTRE LA DÉCOLORATION
OU LE LAVAGE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **31.12.2007 US 18055**

(43) Date of publication of application:
**06.10.2010 Bulletin 2010/40**

(73) Proprietor: **Isp Investments Inc.
Wilmington, DE 19801 (US)**

(72) Inventors:
 • **YAN, Zhou
  Montville, NJ 07045 (US)**
 • **DONNA, Laura, N.
  Nutley, NJ 07045 (US)**
 • **RAYMOND, Rigoletto
  Denville, NJ 07834 (US)**
 • **MOORE, David, J.
  Montclair, NJ 07043 (US)**
 • **FOLTIS, Linda, C.
  Nutley, NJ 07110 (US)**

(74) Representative: **Harrison, Susan Joan et al
Mewburn Ellis LLP
City Tower
40 Basinghall Street
London EC2V 5DE (GB)**

(56) References cited:
 **WO-A2-2006/081496     WO-A2-2007/146672
 US-A1- 2004 052 746     US-A1- 2004 052 746
 US-A1- 2007 041 930     US-A1- 2007 251 026
 US-B2- 7 217 752**

 • **Y Zhou ET AL: "Protection of oxidative hair color
  fading from shampoo washing by
  hydrophobically modifi ed cationic polymers", J.
  Cosmet. Sci, 1 May 2009 (2009-05-01), pages
  217-238, XP055172428, Retrieved from the
  Internet:
  URL:http://journal.scconline.org/pdf/cc200
  9/cc060n02/p00217-p00238.pdf [retrieved on
  2015-02-26]**

**Description**

FIELD OF THE INVENTION

This invention relates to a method of protecting dyed hair color from fading or wash-out, and more particularly, to the use of particular polymers, such as hydrophobically modified polymers, for dyed hair color protection against shampoo treatments.

BACKGROUND OF THE INVENTION

The coloring of hair has become increasingly popular in recent years. However, fading of artificial hair color has become a common problem and a frequent complaint by consumers. Fading can occur during the shampoo washing treatment as color wash-out, or can be initiated by environmental circumstances, such as by exposure to UV radiation. The washing process is the most significant factor in the removal of hair color, while UV exposure had a significant impact only after 90 hours of intense irradiation. S. Marchioretto, "The Use of Silicones as a Color Lock Aid in Rinse-Off Hair Conditioners", J. of Cosmetic Science, 2003 Annual Scientific Meeting, pp. 130-131. Furthermore, the surfactants present in shampoo formulations provide a wetting function which brings moisture into the hair shaft, thus facilitating the removal of the dye molecules to exit during the water rinsing process.

Maintaining hair color and minimizing hair color fading is highly desirable in the hair care market. Several anti-fading products exist in the market including anti-fading shampoos and conditioners. Some products contain silicones such as dimethicone and amodimethicane, which are believed to effect color retention. See A. Schlosser, "Silicones Used in Permanent and Semi-Permanent Hair Dyes to Reduce the Fading and Color Change Process of Dyed Hair Occurring by Wash-Out or UV Radiation", J. Cosmettc Sci., 55 (Supplement), pp.123-131, 2004.

An article published in 2004 in *HAPPI,* relating to the permanency of Level 2 (a.k.a. demi-permanent) and Level 3 (a.k.a. oxidative or permanent) hair dyes, described an olefin graft conditioner ethylene/acrylate copolymer and acrylates amino methacrylate copolymer, at a 2-5% use-level improved color retention (vs. no treatment) by 15% for Level 2 dyes and by 5% for Level 3 dyes when incorporated into a two-part dye rather than applied as a post-dye conditioner.

The proposed mechanism for this function is the formation of an occlusive olefin barrier on the surface of the hair. Martin, T., and Burns, T., "Novel Graft Polymers Boost Hair Color Retention", HAPPI, pp. 92-95, October 2004.

US 2004/0052746 describes multi-purpose vinyl addition polymers with good rheological properties. WO 2007/146672 describes the use of a monoalkyl quaternary ammonium salt, to prevent colour loss from treated hair. US 2007/0041930 describes the use of polysiloxanes which comprise a number of quaternary ammonium groups in the molecule for the cleaning and care of human hair.

U.S. Patents 7,066,966 and 7,147,672 disclose an oxidation dyeing composition for keratin fibers comprising a cationic poly (vinyllactam).

Hydrophobically modified cationic polymers or hydrophobically modified polymers plus a quaternizing agent are effective in protecting dyed hair color from color erosion induced by daily shampooing. The hydrophobically modified cationic polymers or hydrophobically modified polymers plus a quaternizing agent have both hydrophobic moieties and sufficient cationic charge density to maintain substantivity to hair while providing a hydrophobic barrier to "lock-in" hair dye from washing out during daily shampoo washing.

The present invention provides compositions containing a particular terpolymer that protects dyed hair color against shampoo washings. Polymers include one or more polymers selected from the following groups of polymers:

1. Hydrophobically modified quaternary polymers

or

2. Hydrophobically modified polymers plus a cationic surfactant

or

3. Polymers containing diethylanzinopropyl methacrylamide (DMAPMA) or dimethylaminoethyl methacrylamide (DMAEMA) or diethylaminoethyl methacrylamide (DEAEMA)

Hydrophobically modified quaternary polymers include quaternized polymers containing an alkyl substitution with a carbon number above three. The alkyl substitution can be either attached to the quaternary unit or to any other locations of the polymer. The preferred hydrophobically modified quaternary polymers include the quaternary polymers of general formula $(R,R',R'', N-)^+ X^-$, wherein R, R', are identical or different. They can be aliphatic or carry additional substituents. R'' is an alkyl chain with a carbon number above 3, preferably from 8 to 22. $X^-$ represents an anion, for example, chloride, bromide, etc., and N can be part of a heterocyclic or aromatic ring.

The present invention relates to methods of treating hair using the terpolymer of vinylpyrrolidone(VP), dimethylaminopropyl methacrylamide (DMAPMA), and methacryloylaminopropyl lauryldimonium chloride (MAPLDAC) with a trade name of Styleze W-20® Other polymers include, alkyl substituted quatemized cellulose polymers such as those with trade names of Quatrisoft polymer LM-200 (Polyquatemium 24) and SoftCAT™ Polymers such as quatemized hydroxyethyl cellulose polymers with cationic substitution oftrimethyl ammonium and dimethyldodecyl ammonium and stearyldimonium hydroxyl ethyl or propyl cellulose (Crodacel QS) and Cocodimonium hydroxypropyl oxyethyl cellulose (Crodacel QM); alkyl substituted quaternary dimethicone copolymers such as Polyquaternium-41 or 42. However the other polymers mentioned above except for Styleze W-20 are explicitly not part of the inventive treatment method, which is solely as defined in the appended claims and strictly limited thereto.

Hydrophobically modified polymers include polymers containing an alkyl moiety with a carbon number of at least 3. Examples of hydrophobically modified polymers include, but are not limited to nonionic hydrophobically modified polymers such as the copolymer of PEG-150, decyl alcohol and saturated methylene diphenyldiisocyanate with a trade name af Aculyn 44; nonionic guar gum with alkyl groups such as those with trade names of Jaguar HP-60, N-Hance® hydroxypropyl Guar; PVP/Eicosene copolymer; Nonoxynyl hydroxylcellulose with a trade name of Amercell Polymer HM-1500; cetyl hydroxyethyl cellulose (Natrosol Plus); hydroxy propyl Cellulose; hydroxyl propylmethyl cellulose.

Examples of polymers containing dimethylaminopropyl methacrylamide (DMAPMA) or dimethylaminoethyl metharcrylamide (DMAEMA) include poly(vinylpyrrolidone/dimethylaminopropyl methacrylamide) with a trade name of Styleze CC-10 and poly(vinylpyrrolidone/dimethylaminoethyl methacrylate) with a trade name of Copolymer 937 or 845.

The amount of polymer used in the compositions described herein depends upon the particular composition and usage of the composition. Typically, the amount of polymer in accordance with certain aspects of the present invention ranges from about 0.1 to about 10%, preferably from about 0.5 to about 5.0 %, and more preferably from about 1.0 to about 3.0% by weight, based on the total weight of the composition.

The cationic surfactants include quaternized surfactants. The preferred quaternized cationic surfactants are the quaternary ammonium compounds of general formula $(R,R',R'',R'''N)^+ X^-$, wherein R, R', R" and R'" are identical or different ,are aliphatic or carry additional substituents, $X^-$ represents an anion, for example, chloride, bromide, sulfate, etc., and N can be part of a heterocyclic or aromatic ring.

In particularly useful quaternized surfactants, R and R' are $CH_3$, and R" and R'" are aliphatic or aromatic chains, for example, hydroxy ethyl cetearamidopropyldimmonium chloride.

A suitable amount of cationic surfactant ranges from about 0.01 to about 30%, preferably from about 0.01 to about 25%, and more preferably from about 0.2 to about 20% by weight, based on the total weight of the composition.

The color protection treatment herein can be delivered by a post-color treatment (after dying of hair), either from a leave-in product or a rinse-off product or a combination thereof to provide at least 10% color protection against the untreated control after 10 time washes.

For optimum performance, the new hair color should be treated with the leave-in treatment formula and dried before the first shampoo wash and treated with the leave-in formula again after each shampoo and conditioner use.

The post color leave-in treatment formulations preferably may be in the form of a gel or cream or mousse or spray. The post color rinse off treatment typically can be in the form of shampoos, conditioners or other rinse off product forms.

Test methods for evaluating hair color changes for anti-fading effect

Polymer Screening Test - Soaking Test of Dyed Hair

1. Hair dye dissolution in water was determined by soaking fixed amount of dyed hair sample (0.5g) in water containing 1% polymer (as solid). The soaking liquor was sampled after fixed hours of soaking and read for L, a and b values on HunterLab colorimeter (t=0 values were read before adding hair into a soaking liquor). Color changes generated by hair dye dissolution in soaking liquor were determined as dE calculated from L, a, b values of the soaking liquor before and after soaking.

$$dE = ((L_t - L_o)^2 + (a_t - a_0)^2 + (b_t - b_o)^2)^{1/2}.$$

Where $L_o$, $a_o$, $b_o$ ; and $L_t$, at , $b_1$ are measured Hunter L, a, b color parameters before and after soaking at certain time period, respectively.

A larger value of dE reflects greater change of color.

*Fading Index = dE of polymer solution / dE of water.* Maximum Fading index is 1, indicating no protection at all.

Polymers showing reduced dye dissolution or with a Fading Index less than 0.5 are further tested by multiple shampoo washing tests.
2. Multiple Shampoo Washing Test of Dyed Hair Tresses - Color Fastness Test

Polymers which show effectiveness in reducing hair dye dissolution in the soaking tests were selected to test in a full hair cream or gel formulations in multiple shampoo washing test. These polymers were formulated into hair care formulations as either a leave in treatment or rinse off formulations.

Hair samples

Bleached hair was purchased from International Hair Importers. Each hair tress weighs 3.5g and has a 1.5" width and a 6.5" length. It was split into halves for color fastness test, one half for the treatment test and the other half served as a non-treated control.

Hair dyes

The bleached hairs were dyed using commercially available hair coloring products. The hair dye product tested in accordance with the certain examples were ones from red shade containing -Hydroxyethyl 4,5-Diamino Pyrazole Sulfate, which is thought to be the most delicate dye. One is intense dark red and the other is radiant Ruby. Intense dark red is used in most tested hair samples unless specified. In addition, a dark brown color of commercial hair coloring product was also used in some tests.

10x washing and treatment procedures

The wax tab of a hair tress was cut into two separate parts. One half was used as the control test, non-treatment tress. The other half was used for treatment test. The control half was washed with 0.75g 12% SLES solution for 2min., with one minute shampooing with rubbing and one minute rinse under running warm tap water. Then the hair tress was dried with a cold air hair blow dryer. The wash cycle was repeated for 10 times. Three different treatment procedures were used, leave-in treatment, rinse off treatment and the combination of leave-in and rinse off treatment. For a leave-in treatment test, 0.30g product was applied to a damp, one half of dyed hair tress before the first shampoo, massaged gently through the tress and dried with cold air hair drier, followed by washing with 12% SLES then treated again with the leave-in formulation and cold air blow dried. Then the SLES wash and treatment and blow dry cycle were repeated 10 times. For a rinse off treatment, hair tress was washed by the treatment shampoo and/or treatment conditioners, cold air blow dried and repeated 10 cycles against 12% SLES as a control. For the combination of leave-in and rinse off treatment, the damp hair tress was treated with 0.30g product, massaged gently through the tress and dried with cold air hair drier, followed with wash by the treatment shampoo and/or treatment conditioners. The cycle was repeated 10 times.
At the end of 3x, 5x, 8x and 10x washes, the dried hair tresses were measured for L, a, values using a HunterLab colorimeter and dE was calculated as color changes after washes.

Color analysis of hair tresses before and after multiple shampoo washes:

Hair color changes before and after washes were determined by dE.
Color changes were measured by measuring Hunter L, a and b values on a HunterLab colorimeter. dE was calculated using the following equations to evaluate color change before and after washes.

$$dE = ((L_t\text{-}L_o)^2 + (a_t\text{-}a_0)^2 + (b_t\text{-}b_o)^2)^{1/2} \cdot$$

Where $L_o$, $a_o$, $b_o$: and $L_1$, $a_t$, $b_t$ are measured Hunter L, a, b color parameters before and after washing, respectively.
The larger value of dE reflects greater change of color. It is reported that color difference with dE greater than 1 are generally perceptible by eye.

$$\% \text{ Color Protection} = \% \text{ dE improvement} = (dE \text{ treatment} - dE \text{control}) \times 100 / dE\text{control}$$

A % dE improvement or % Color Protection over 10-15% is perceivable by eye.

## EXAMPLE 1

Table 1 shows the results of color change of soaking liquor from dyed hair samples soaked in different polymer aqueous solutions (1% active), expressed as a Fading Index.

**Table 1**

| Polymer No. | Polymer trade name | INCI name | Soaking test Fading index* | Soaking test Fading index* |
|---|---|---|---|---|
| | | | 30min. | 90min. |
| 1 | Styleze W-20 | Terpolymer of vinylpyaolidone(VP), dimethylaminopropyl methacrylamide (DMAPMA), and methacryloylaminoptopyl lauryldimonium chlaride (MAPLDAG) | 0.11 | 0.35 |
| 2 | Styleze CC-10 | Poly(vinylpyrrolidone/dimethylaminopropyl methacrylamide)(DMAPMA) | 0.33 | 0.31 |
| 3 | Conditioneze NT-20 | Polyquaternium-28, Polyvinyl pyrrolidone and methacryamidopropyl trimethylammonium chloride (MAPTAC) | 0.71 | 0.84 |
| 4 | Copolymer 937 | Poly(vinylpyrrolidone/dimethylaminoethyl | 0.34 | 0.46 |
| | | methacrylate)(DMAEMA) | | |
| 5 | Gafquat 755N | Quatemized copolymers of vinylpyrrolidone and dimethylaminoethyl methacrylate | 0.73 | 0.94 |
| 6 | Aquaflex FX-64 | Isobutylene/ethylmaleimide/hydroxyethylmaleimide | 0.63 | 1 |

FI = Fading Index = dEpolyrner/dEwater, maximum = 1 = maximum fading.
*A Fading Index less than 0.5 for a polymer is considered to be project grant effective and is as supported also by multiple data in a shampoo wash test.

The results in Table 1 show that polymer #1 within the scope of this invention containing both a hydrophobic chain and a quaternary unit is the most effective in reducing hair dye dissolution in the critical first 30 min. soaking time. Polymers # 2 & #4 containing dimethylaminopropyl methacrylamide (DMAPMA) and dimethylaminoethyl methacrylate)(DMAEMA) are within the scope of this invention and are effective as well. By contrast, comparative examples with polymers #3, 5, 6, are ineffective in reducing hair dye dissolution.

Table 1a provides results of a soaking test with hydrophilic polymers (PVP) which also fail to provide the fade resistance obtainable with certain aspects of the present invention. The results indicate the ineffectiveness of these polymers in reducing hair dye dissolution in water.

Table 1a

| Hydrophilic polymers | FI @30min. | FI @90min. |
|---|---|---|
| PVP K 90 | 0.89 | 0.95 |
| PVP K 120 | 0.71 | 0.99 |
| PVP K 15 | 1.03 | |
| PVP K30 | 1.03 | |
| PVP K 60 | 1.01 | |
| PVPNA | 1.03 | |

As comparative examples, other polymers were tested in both the soaking test and multiple shampoo washes. The results are given in Table lb. Both the soaking and multiple shampoo wash tests demonstrate the ineffectiveness of these polymers.

Table 1b.

| Polymers | INCI name | FI | FI | 10x wash test |
|---|---|---|---|---|
| | | 30min. | 90min. | |
| Cotton bloom | Oligosaccharides | 1.14 | 1.57 | negative |
| Aquaflex XL-30 | Copolymer of Isobutylene/dimethylaminopropyl malcimide/othoxylated malcimide/mateic acid copolymer | 1.27 | 1.13 | no effect |
| Styleze 2000, pH7, anionic polymer | Copolymer of vinyl pyrrolidone (VP)/Acrylates/Lauryl Methacrylate | 0.82 | 0.80 | negligible effect, |
| Chitosan | Chitosan | | | negative |
| PVA/VAM | Coplymer of vinyl alcohol and vinyl amide | 1.01 | 1.32 | |
| PVA MW 85K | Poly vinyl alcohol | 0.94 | 1.23 | |
| PVA MW 31K | Poly vinyl alcohol | 1.15 | 1.41 | |
| Aquaflex XL-30 +Ultrathix P 100 | Ultrathix P 100 is cross linked acrylate polymer | | | negligible effect |
| XL-30 +Gantrez P 904 | Copolymer of vinyl pyrrolidone and-long chain a-olefin | | | ineffective |
| Hydroxypropyl quar without a quatemizing agent) | Hydroxypropyl quar gum | | | no effect |

EXAMPLES 2-9

Polymer Nos. 1, 2 and 4 from Example 1, which showed effectiveness in reducing hair dye dissolution in water, were tested in a clear gel formulation in a multiple shampoo washing test following the procedure described in the test method section. The hair tresses were treated with the anti-fading gel first, cold air blow dried and then washed with 12% SLES. The treatment and wash cycle was repeated 10x. As a control, the hair tress was treated with the same formulation without the polymer, 11506-85, then blow dried and washed with SLES. The test results are shown in Table 3.

Table 2 Gel formulation containing test polymers for leave in treatment in a 10x washing test.

| | Test formula 11506-85A - F; 56B, 102 -111; 10748-67 | Control formula 11506-85 |
|---|---|---|
| Ingredient | wt% | wt% |
| Jaquar HP-60, Hydroxy propyl Guar gum (as a thickner) | 1.5 | 1.5 |
| Test polymer (Nos. 1, 2, or 4) | 2.0% (active) | 0 |
| Glycerin | 1.0 | 1.0 |
| Liquid Gernmal Plus | 0.5 | 0.5 |
| Water | To 100 | To 100 |

Table 3. Hair color fastness test results, %Protection = %dE = (dE treatment -decontrol) x100 / dEcontrol

| Example # | Testing polymer/ Formula# | Structure unit | Tress# | 3x wash %dE | 5x wash %dE | 8x wash %dE | 10x wash %dE |
|---|---|---|---|---|---|---|---|
| 2 | Copolymer 937/11506-85A | VP-DMAEMA | 58H | 19 | | | 7 |
| 3 | Styleze CC-10/11506-56B | VP-DMAPMA | 53H | 18 | 7 | 5 | 14 |
| 4 | Styleze W-20 /10748-67 | VP-DMAPMA - C12 quat | 35H | 27 | 21 | 24 | 38 |
| 5 | Conditioneze NT-20/85B | VP-MAPTAC(quat) | 59H | 6 | 9 | -5 | -6 |

The multiple shampoo washing test results in Table 3 demonstrate that polymers within the scope of this invention contain both a hydrophobic chain and a quaternary unit. Styleze W-20 (Example4) provides the best color protection benefit. Consistent with the soaking test result in Table 1, a quaternary polymer, Conditioneze NT-20 (Example 5)(comparative), containing ne hydrophobic chain and no DMAPMA or DMAEMA unit is ineffective.

Another chemical class of polymer, hydroxyl ethyl cellulose (HEC) modified with either an alkyl chain (hydrophobicity) and/or quaternization, was tested for anti-fading effect in the multiple shampoo washing tests. These polymers were formulated into the same test formula as specified in Table 2, except Natrosol Plus (alky hydroxyl cellulose) in which Jaquar HP-60, Hydroxy propyl Guar gum, was not added . The multiple shampoo washing test results are given in Table 4 below.

Table 4. Hair color fastness test results, %Protection (%dE) = (dE treatment -dEcontrol) x 100 / dEcontrol

| Examples # | Testing polymer/ Forrnula# | Structure unit | Tress# | 3x wash %dE | 5x wash %dE | 8x wash %dE | 10x wash %dE |
|---|---|---|---|---|---|---|---|
| 6 | Cetyl-Hydroxy Ethyl Cellulose (HEC), Natrosol Plus / 11506-106 | Hydrophobic modified cellulose | 78H | 0 | 13 | 9 | 15 |
| 7 | Quaternary salt of HEC, Palyquaternium - 10, Ritaquta 400LR/ 11506-105 | Quaternized HEC | 77H | 5 | 5 | 19 | 16 |
| 8 | C12 -HEC quaternary, Polyquaternium -24, Quatrlsoft polymer LM-200/11506-102 | C12- HEC quat | 76H | 11 | 22 | 25 | 21 |
| 9 | Steardimonium Hydroxyethyl Cellulose, Crodacel QS/11506-111 | C18-HEC quat | 95H | 18 | 17 | 19 | 31 |

The results in Table 4 demonstrate that the HEC derivatives containing both hydrophobic and quaternary units (Examples 8 and 9) provide acceptable levels of hair color fastness, especially in the important early washing stages.

## EXAMPLE 10

A comparative composition containing an anionic polymer with a hydrophobic chain was tested. The anionic polymer was Styleze 2000, a copolymer of vinyl pyrrolidone (VP)/Acryates/Lauryl Methacrylate, neutralized and formulated into the same test formulation and tested using the procedure specified in Examples 2-9. The results of the color fading test of this anionic hydrophobic polymer are given in Table 5 which shows that its effect on anti-fading is negligible or minor (<10%). The result from the soaking test is also listed in Table 5. Test results for the cationic hydrophobic polymer, Styleze W-20 is also listed showing the improved results obtained with the present invention.

Table 5 Hair color fastness test results, %protection , %dE = (dE treatment -dEcontrol) x100 / dEcontrol

| Hydrophobic polymer / Formula# | FI@ 30m | FI@ 90m | Tress# | 3x wash %dE | 5x wash %dE | 8x wash %dE | 10x wash %dE |
|---|---|---|---|---|---|---|---|
| Styleze 2000 (anionic & C12 chain polymer) /11506-110 | 0.82 | 0.80 | 79H | 14 | 15 | 7.8 | 7.2 |
| Styleze W-20 (VP-DMAPMA - C12 quat) /10748-67 | 0,11 | 0.35 | 35H | >50 | 38 | 15 | 37 |

## EXAMPLE 11

This example shows the anti-fading effect of hydrophobically modified polymers plus a quaternizing agent. Aculyn 44 is a hydrophobically modified nonionic polymer and was formulated into a hair care leave in treatment together with a cationic surfactant, Hydroxyethyl Cetearamidopropyldimonium Chloride in a raw material with a trade name of Prolipid 161®. The leave in treatment formulation is listed in Table 6. The color fading test results are listed in Table 7.

Table 6 Formulation of leave in hair treatment, 11337-82B

| Item# | Ingredients (Trade)/ Supplier | INCI | Wt.% |
|---|---|---|---|
| Phase A 1 | Water | Water | 84.56 |
| 2 | Dissolvene NA-2 Hampshire | Disodium EDTA | 0.20 |
| 3 | Propylene Glycol | Propylene Glycol | 1.00 |
| 4 | Aculyn 44 (@ 1% active) Rohm & Haas | PEG-150 Decyl Alcohol/SMDI Coplymer (34.85% actives) | 2.87 |
| Phase B 5 | Prolipid® 161 (0.86% active)ISP | Hydroxyethyl Cetearamidopropyldimonium Chloride (and) Behenyl Alcohol (and) Cetearyl Alcohol | 2.87 |
| 6 | Jeecol-20 Jeen | Steareth-20 (HLB 15.3) | 0.40 |
| 7 | Jeecol-2 Jeen | steareth-2 (HLB 4.9) | 0.60 |
| 10 | Lanette O Wax Cognis Care | Cetyl Stearyl Alcohol | 2.00 |
| Phase C 11 | Water | Water | 5.00 |
| 12 | Liquid Germall Plus ISP | Propylene Glycol (and) Diazolidinyl Urea (and) 0.50 Iodopropynyl Butylrarbamate | 0.50 |

Color fastness test results in Table 7 demonstrate that nonionic hydrophobically modified polymer plus cationic surfactant in Prolipid 161 provided significant benefit for anti-fading of hair color while either of them alone is ineffective by comparing test# 11-2 (inventive) to test# 11-1 and 11-3 (comparative).

Table 7 Hair color fading test results, %Protection = %dE = (dE treatment -dEcontrol) x 100 / dEcontrol

| Test # | Testing polymer/ Formula# | INCI name | Tress# | 3x wash %dE | 5x wash %dE | 8x wash %dE | 10x wash %dE |
|---|---|---|---|---|---|---|---|
| 11-1 | Aculyne 44 in Jaguar HP-60 gel/11506-85C | PEG-150 Decyl Alcohol/SMDI Copolymer | 67H | 5 | 0 | 0 | 2 |
| 11-2 | Aculyn 44 in Prolipid 161 cream/11337-82B | | 26H | | 36 | 41 | 48 |

(continued)

| Test # | Testing polymer/ Formula# | INCI name | Tress# | 3x wash %dE | 5x wash %dE | 8x wash %dE | 10x wash %dE |
|---|---|---|---|---|---|---|---|
| 11-3 | Prolipid 161 in water | Hydroxyethyl Cetearamidopropyldimonium Chloride (and) Behenyl Alcohol (and) Cetearyl Alcohol | 7H | | | | 0 |

Color fastness test results in Table 8 further prove that the anti-fading effect in the leave in treatment formula, 11337-82B, is attributable to the synergistic effect of hydrophobic polymer, Aculyn 44 and the quaternizing agent content in Prolipid 161.

Table 8 Color fading test results after 10x washes, %Protection = %dE = (dE treatment, - dEcontrol) x100 / dEcontrol

| Test # | 11-4 | 11-5 | 11-6 | 11-7 |
|---|---|---|---|---|
| Tress# | 4H, 8H, 26H | 7H | 5H | 3H |
| Formula Discription | Original 11337-82B | Prolipid + Jeecols 11337-87A | Aculyn 44 + Jeecols 11337-84 | Aculyn 44 +water 11337-85 |
| Ingredients | | | | |
| Water | 84.56 | 87.43 | 92.43 | 96.43 |
| Disodium EDTA | 0.20 | 0.20 | 0.20 | 0.20 |
| Propylene Glycol | 1.00 | 1.00 | 1.00 | 0.00 |
| **Aculyn 44** | **2.87** | **0.00** | **2.87** | **2.87** |
| **Prolipid 161** | **2.87** | **2.87** | **0.00** | **0.00** |
| Jeecol-20 Steareth-20 | 0.40 | 0.40 | 0.40 | 0.00 |
| Jeecol-2 steareth-2 | 0.60 | 0.60 | 0.60 | 0.00 |
| Cetyl Stearyl Alcohol | 2.00 | 2.00 | 2.00 | 0.00 |
| Water | 5.00 | 5.00 | 0.00 | 0.00 |
| Liquid Germall Plus | 0.50 | 0.50 | 0.50 | 0.50 |
| **Color fastness test results, %dE improvement** | **20%, 25%, 48%** | **0%** | **0%** | **0%** |

## EXAMPLE 12

Table 10 shows the color fastness results of a leave in treatment formula containing hydrophobic polymer, Aculyn 44, quaternizing agent content from Prolipid 161 and hydrophobic quaternary polymer, Styleze W-20. The formula ingredients are listed in Table 9. The daily leave in treatment formula provides 37% and 44% color protection with the two types of dye tested.

Table 9 Formulation of Leave in treatment cream, 11261-20

| Item# | Ingredients (Trade) Supplier | INCI | Wt.% |
|---|---|---|---|
| Phase A 1 | Water | Water | 79.62 |
| 2 | Dissolvene NA-2 Hampshire | Disodium EDTA | 0.20 |
| 3 | Propylene Glycol | Propylene Glycol | 1.00 |

(continued)

| Item# | Ingredients (Trade) Supplier | INCI | Wt.% |
|---|---|---|---|
| 4 | Aculyn 44 (use @ 1% active) Rhom & Haas | PEG-150 Decyl Alcohol/SMDI Coplymer (34.85% actives) | 2.87 |
| Phase B 5 | Prolipid® 161 (using 0.86% active)ISP | Hydroxyethyl Cetearamidopropyldimonium Chloride (and) Behenyl Alcohol (and) Cetearyl Alcohol | 2.87 |
| 6 | Jeccol-20 Jeen | Steareth-20 | 0.40 |
| 7 | Jeecol-2 Jeen | steareth-2 | 0.60 |
| 8 | Ceraphyl SLK ISP | Isodecyl Neopentanoate | 2.00 |
| 9 | Beauty Butter ISP | Shea Butter | 0.50 |
| 10 | Lanette O Wax Cognis Care | Cetyl Stearyl Alcohol 2.00 | 2.00 |
| Phase C | Water | Water | 5.00 |
| 12 | Styleze W-20 (20.5 % active - use @ 0.5%)ISP | Polyquaternium-55 | 2.44 |
| 13 | Liquid Germall Plus ISP | Propylene Glycol (and) Diazolidinyl Urea (and) Iodopropynyl Butylcarbamate | 0.50 |

Table 10 Hair color fading test results of formula 11261-20, %Protection = %dE = (dE treatment -dEcontrol) x100 / dEcontrol

| Test# | Testing dye | Tress# | 3x wash %dE | 5x wash %dE | 8x wash %dE | 10x wash %dE |
|---|---|---|---|---|---|---|
| 12-1 | Intense dark red | 21H | 11 | 20 | 32 | 37 |
| 12-2 | Radiant Ruby | 20H | 32 | 42 | 39 | 44 |

## EXAMPLE 13

To further demonstrate the synergistic effect of polymer with a hydrophobic unit and a quat, hydroxypropyl quar gum which contains propyl unit was tested together with Prolipid 161 in color fastness test. Table 11 listed the test formula and the control test formula.

Table 11 Gel formula containing testing polymers for leave in treatment in10x washing test.

| | Test formula 11506-117 | Control formula 11506-117a | Test formula 11506-48 |
|---|---|---|---|
| Ingredient | wt% | wt% | wt% |
| Jaquar HP-60, Hydroxy propyl Guar gum (as a thickener) | 2.0 | 0 | 1.5 |
| Prolipid 161, Hydroxyethyl Cetearamidoprogyldimonium Chloride (and) Behenyl Alcohol (and) Cetearyl Alcohol | 3.0 | 3.0 | 0 |
| Liquid Gemmal Plus | 0.5 | 0.5 | 0.5 |
| Water | To 100 | To 100 | To 100 |

The color fastness results of the test formula and the control formula in Table 11 are shown in Table 12. Treatment with formula 11506-117 containing hydroxy propyl quar and Prolipid 161 which is within the scope of this invention provides 19% color protection after 10x washes (test# 13-2) while the quar gum alone (Test# 13-1) and Prolipid 161 alone (Test# 13-3) are ineffective after 10x washes.

Table 12 Hair color fading test results of formula 11506-117, %Protection = %dE = (dE treatment dEcontrol) x100 / dEcontrol

| Test # | Testing formula | Tress# | 3x wash %dE | 5x wash %dE | 8x wash %dE | 10x wash %dE |
|---|---|---|---|---|---|---|
| 13-1 | 11506-48 vs untreated | 34H | 17 | 13 | 1 | 0 |
| | | | | | | |
| 13-2 | 11506-117 vs 117a | 91H | | 20 | 21 | 19 |
| 13-3 | 11337-117 vs untreated | 7H | | | | 0 |

**EXAMPLE 14**

The polymer within the scope of this invention contain both a hydrophobic chain and a quaternary unit, Styleze W-20® was formulated into a rinse off formula, a 2-in-1 shampoo formulation and a conditioner formulation to test its color fastness effect. The 2-in-1 shampoo and conditioner formulations are listed in Table 13 & 14. The rinse off formulations were either tested alone or together with a leave in gel formulation (Table 15) as specified bellow.

1. Styleze W-20® 2-in-1 shampoo wash versus 12% SLES wash as a control, 10 cycles

2. Styleze Vsl-20® 2-in-1 shampoo wash versus the control with the 2-in-1 shampoo wash without Styleze W-20® in it, 10 cycles

3. First treatment to the hair with the leave in treatment formula, 10748-67, blow dried, followed by Styleze W-20® 2-in-1 shampoo wash vs the control with no Styleze W-20® in the leave in gel treatment, 11506-85 and SLES washes, 10 cycles.

4. First treatment to the hair with the leave in treatment formula, 10748-67, blow dried, followed by Styleze W-20® 2-in-1 shampoo wash and using Styleze W-20® conditioner and rinse vs the control with treatment containing no Styleze W-20® leave in gel treatment, 11506-85 and SLES washes, followed with application of no Styleze W-20® conditioner and rinse, 10 cycles.

Table 13 2-in-1 Shampoo formula, Pearlescent Bodifying Shampoo

| Ingredients | 10906-131A, wt% | Control, 11571-7, wit% |
|---|---|---|
| Water | 55.73 | 58.23 |
| **Stylize W20 (20% active)** | 2.5 | 0 |
| ocoamphodiacetate(Miranol C2M NP) | 15.0 | 15.0 |
| Cocamidopropyl Betaine (Miratain CB) | 3.52 | 3.52 |
| Ammonium Lauryl Sulfate (Standapol A) | 6.0 | 6.0 |
| Sodium Lauroyl Sarcosinate(Maprosyl 30) | 12.0 | 12.0 |
| C12-C15 Alktl Lactate (Ceraphyl 41) | 0.50 | 0.50 |
| Glycol Stearate(Cerasynt IP) | 0.25 | 0.25 |
| Liquid Germal Plus | 0.30 | 0.30 |
| Sodium Chloride(25% solution) | 2.00 | 2.00 |
| Citric acid(25% solution) | 2.20 | 2.20 |

Table 14 Conditioner formula

| Ingredients | 11571-6A wt% | 11571-6B wt% | Supplier |
|---|---|---|---|
| Water | 89.90 | 92.90 | |
| Disodium EDTA (Versene NA2) | 0.10 | 0.10 | |
| Proltpid®161 | 2.50 | 0 | ISP |
| Cetearyl Alcohol (Lanette O) | 3.00 | 3.00 | Carc Chemicals : |
| Glyceryl Stearate (and) PEG-100 Stearate (Arlacel 165) . | 1.50 | 1.50 | Uniqema/ICI |
| Polyquaternium-55 (Styleze® W-20), 20% active | 2.50 | 2.50 | ISP |
| Propylene Glycol (and) Diazolidinyl Urea (and) Iodopropynyl Butylcarbamate (Liquid Germall® Plus) | 0.50 | 0.50 | ISP |
| Citric Acid (50% aqueous solution) | 0.35 | 0.35 | |

Table 15 Leave in gel formula containing Styleze W-20

| | Test formula 10748-67 | Control formula 11506-85 |
|---|---|---|
| Ingredient | wt% | wt% |
| Jaquar HP-60, Hydroxy propyl Guar gum (as a thickner) | 1.5 | 1.5 |
| Testing polymer | 2.0% (active) | 0 |
| Glycerin | 1.0 | 1.0 |
| Liquid Gernmal Plus | 0.5 | 0.5 |

Table 16 shows the color fastness test results of dyed hair treated with Styleze W-20 rinse off, leave in treatment or combination of both. Three commercial products were also tested using the same test procedure for comparison. The results in Table 15 demonstrate that Styleze W-20 in the 2-in-1 shampoo formula (10906-131A) provides 22% protection at the end of 10 washes (compare test# 14-1a, 14-1b with14-1c), which is much better than the two commercial products (test # 14-6 & 14-7). The combination treatment from the leave in gel, 10748-67, and the 2-in-1 shampoo provides the highest color protection, 45% to 51 % at the end of 10 washes (test# 14-2a and 14-2b), which is better than the commercial product 1 (test# 14-5) and significantly more effective than commercial product 2 & 3 (test # 14-6 & 14-7). Test 14-7 and 14-8 are the tests of W-20 leave in gel, 2-in-1 shampoo and conditioner against commercial color lock product 2 as the control using red hair color samples and dark brown dyed hair respectively. Both results show the W-20 leave in and rinse off treatment are about 40% superior than the commercial product.

Table 16 color fading test results, %Protection = %dE = (dE treatment -dEcontrol) x100/ dEcontrol

| Test # | Formula# | Treatment types | Tress# | 3x wash %dE | 5x wash %dE | 8x wash %dE | 10x wash %dE |
|---|---|---|---|---|---|---|---|
| 14-1a | 10906-131A vs SLES | W-20 2-in-1 Shampoo | 56H | 29 | 9.7 | 33 | 22 |
| 14-1b | 10906-131A vs SLES | W-20 2-in-1 Shampoo | 75H | 15 | 15 | 31 | 20 |
| 14-1c | 10906-131A vs 11571-7 | W-20 2-in-1 Shampoo vs no W 20 shampoo | 63H | 9 | 15 | 30 | 20 |
| 14-2a | 10748-67 + 10906-131a vs 11506-85 & SLES | Leave in gel + W-20 2-in-1 Shampoo | 73 H | 36 | 40 | 44 | 45 |
| 14-2b | 10748-67 + 10906-131a vs 11506-85 & SLES | Leave in gel + W-20 2-in-1 Shampoo | 81H | 44 | 49 | 45 | 51 |
| 14-3 | 11571-6a vs 11571-6b SLES washes | W-20 conditioner vs no W-20 cond. | 57H | 11 | 9 | 12 | 7 |

(continued)

| Test # | Formula# | Treatment types | Tress# | 3x wash %dE | 5x wash %dE | 8x wash %dE | 10x wash %dE |
|--------|----------|-----------------|--------|-------------|-------------|-------------|--------------|
| 14-4 | 10748-67 + 10906-131a &11571-6a against the control | Leave in gel + W-20 2-in-1 Shampoo + W 20 conditioner | 74H | 0 | 8 | 17 | 24 |
| 14-5 | Commercial color lock product 1 vs SLES washes | Leave in cream + shampoo + conditioner | 83H | 19 | 20 | 35 | 39 |
| 14-6 | Commercial color lock product 2 vs SLES washes | Shampoo + conditioner | 89H | 0 | 5 | 8 | 11 |
| 14-7 | Commercial color lock product 3 vs SLES washes | Shampoo + conditioner | 90H | 41 | 24 | 3 | 12 |
| 14-8* | 10748-67 + 10906-131a &11571-6a against commercial color lock product 2 | Leave in gel + W-20 2-in-1 Shampoo + W 20 conditioner | 96H | | 26 | 36 | 40 |
| 14-9** | 10748-67 + 10906-131a &11571-6a against commercial color lock product 2 | Leave in gel + W-20 2-in-1 Shampoo + W 20 conditioner | 100H | 14 | 27 | 20 | 38 |
| * Hair tress was dyed with intense dark red color **Hair tress was dyed with dark brown color | | | | | | | |

## EXAMPLE 15

This example demonstrates the importance of applying leave in treatment to the new hair color before first shampoo washes. Two different tests were conducted. First, newly dyed hair samples were treated with leave in formula, 10748-67, blow dried with cold air, followed by 12% SLES wash. The treatment, dry and wash cycle was repeated 10 time. In another test, newly dyed hair sample was washed with SLES first then treated with formula 10748-67, dried. This cycle was repeated by 10x. The results in Table 17 indicate that applying a leave in treatment before the first shampoo wash of new color is important, providing important color protection during early washes (compare test#15-1 & 2 with test#15-3).

Table 17 Hair color fading test results of formula 10748-67 vs control formula 11506-85, SLES washes , %Protection = %dE = (dE treatment -dEcontrol) x100 / dEcontrol

| Test# | Formula# | Treatment types | Tress# | 3x wash %dE | 5x wash %dE | 8x wash %dE | 10x wash %dE |
|-------|----------|-----------------|--------|-------------|-------------|-------------|--------------|
| 15-1 | 10748-67 vs 11506-85 | Leave in gel before 1st SLES wash | 33H | >50 | 38 | 15 | 37 |
| 15-2 | 10748-67 vs 11506-85 | Leave in gel before 1st SLES wash | 35H | 26 | 20 | 34 | 38 |
| 15-3 | 10748-67 vs 11506-85 | SLES wash first then leave in treatment | 72H | 0 | 19 | 30 | 39 |

## Claims

1.    A post-colour treatment method of protecting dyed hair color from fading or wash-out during exposure to air and/or shampooing which comprises treating said dyed hair in a leave-in manner with a composition which comprises a terpolymer of vinyl pyrrolidone (VP), dimethylaminopropyl methacrylamide (DMAPMA) and methacryloylaminopropyl lauryldimonium chloride (MAPLDAC).

2. The method of claim 1 wherein the terpolymer is present in the composition in an amount of 0.1-10% by weight.

3. The method of claim 2 wherein the terpolymer is present in the composition in an amount of 0.1-3% or 1-3% by weight.

4. A method according to claim 1 wherein said composition is applied to the dyed hair in a leave-in manner prior to first shampooing or rinsing and the leave-in treatment is repeated after each shampooing or rinsing.

5. A method according to claim 1 wherein said polymer is included in the shampoo or conditioner used in the subsequent shampoo treatments.

6. A method according to claim 1 wherein said composition does not contain a dye.

7. A method according to claim 1 wherein said leave in treatment formulation is a gel, cream, mousse or spray.

8. A method according to claim 5 wherein said shampoo includes a conditioner.

**Patentansprüche**

1. Farb-Nachbehandlungsverfahren zum Schutz von kolorierter Haarfarbe vor Ausbleichung oder Auswaschung beim Aussetzen gegenüber Luft und/oder beim Schamponieren, umfassend das Behandeln des kolorierten Haars auf Leave-in-Art mit einer Zusammensetzung, die ein Terpolymer aus Vinylpyrrolidon (VP), Dimethylaminopropylmethacrylamid (DMAPMA) und Methacryloylaminopropyllauryldimoniumchlorid (MAPLDAC).

2. Verfahren nach Anspruch 1, wobei das Terpolymer in der Zusammensetzung in einer Menge von 0,1 bis 10 Gew.-% vorhanden ist.

3. Verfahren nach Anspruch 2, wobei das Terpolymer in der Zusammensetzung in einer Menge von 0,1 bis 3 Gew.-% oder 1 bis 3 Gew.-% vorhanden ist.

4. Verfahren nach Anspruch 1, wobei die Zusammensetzung vor einem ersten Schamponieren oder Spülen auf das kolorierte Haar auf Leave-in-Art angewendet wird und die Leave-in-Behandlung nach jedem Schamponieren oder Spülen wiederholt wird.

5. Verfahren nach Anspruch 1, wobei das Polymer in dem Shampoo oder Konditionierer, das/der in den nachfolgenden Shampoobehandlungen verwendet wird, enthalten ist.

6. Verfahren nach Anspruch 1, wobei die Zusammensetzung keine Kolorierung umfasst.

7. Verfahren nach Anspruch 1, wobei die Leave-in-Behandlungsformulierung ein Gel, eine Creme, ein Schaum oder ein Spray ist.

8. Verfahren nach Anspruch 5, wobei das Shampoo einen Konditionierer umfasst.

**Revendications**

1. Procédé de traitement post-coloration consistant à protéger une teinture pour cheveux contre la décoloration ou le lavage au cours de l'exposition à l'air et/ou au shampooing qui comprend le traitement desdits cheveux teintés d'une manière sans rinçage à l'aide d'une composition qui comprend un terpolymère de vinylpyrrolidone (VP), de méthacrylamide de diméthylaminopropyle (DMAPMA) et de chlorure de méthacryloylaminopropyl lauryldimonium (MAPLDAC).

2. Procédé selon la revendication 1 dans lequel le terpolymère est présent dans la composition en une quantité de 0,1 à 10 % en poids.

**3.** Procédé selon la revendication 2 dans lequel le terpolymère est présent dans la composition en une quantité de 0,1 à 3 % ou de 1 à 3 % en poids.

**4.** Procédé selon la revendication 1 dans lequel ladite composition est appliquée sur les cheveux teintés d'une manière sans rinçage avant le premier shampooing ou rinçage et le traitement sans rinçage est répété après chaque shampooing ou rinçage.

**5.** Procédé selon la revendication 1 dans lequel ledit polymère est incorporé dans le shampooing ou le conditionneur utilisé dans les traitements de shampooing ultérieurs.

**6.** Procédé selon la revendication 1 dans lequel ladite composition ne contient pas de teinture.

**7.** Procédé selon la revendication 1 dans lequel ladite formulation de traitement sans rinçage est un gel, une crème, une mousse ou un spray.

**8.** Procédé selon la revendication 5 dans lequel ledit shampooing comprend un conditionneur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040052746 A
- WO 2007146672 A
- US 20070041930 A
- US 7066966 B
- US 7147672 B

**Non-patent literature cited in the description**

- **S. MARCHIORETTO.** The Use of Silicones as a Color Lock Aid in Rinse-Off Hair Conditioners. *J. of Cosmetic Science, 2003 Annual Scientific Meeting,* 130-131
- **A. SCHLOSSER.** Silicones Used in Permanent and Semi-Permanent Hair Dyes to Reduce the Fading and Color Change Process of Dyed Hair Occurring by Wash-Out or UV Radiation. *J. Cosmettc Sci.,* 2004, vol. 55, 123-131
- **MARTIN, T. ; BURNS, T.** Novel Graft Polymers Boost Hair Color Retention. *HAPPI,* October 2004, 92-95